(19) Europäisches Patentamt
European Patent Office
Office européen des brevets

(11) **EP 2 793 015 A1**

(12) **EUROPEAN PATENT APPLICATION**
published in accordance with Art. 153(4) EPC

(43) Date of publication:
**22.10.2014 Bulletin 2014/43**

(51) Int Cl.:
***G01N 21/27*** (2006.01)

(21) Application number: **13749756.6**

(22) Date of filing: **13.02.2013**

(86) International application number:
**PCT/JP2013/053321**

(87) International publication number:
**WO 2013/122072 (22.08.2013 Gazette 2013/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.02.2012 JP 2012028231**

(71) Applicant: **National University Corporation Tokyo Medical and Dental University Bunkyo-ku Tokyo 113-8510 (JP)**

(72) Inventors:
• **SAKOTA, Daisuke**
  **Tokyo 113-8510 (JP)**
• **TAKATANI, Setsuo**
  **Tokyo 113-8510 (JP)**

(74) Representative: **Cabinet Plasseraud**
**52, rue de la Victoire**
**75440 Paris Cedex 09 (FR)**

(54) **METHOD AND DEVICE FOR MEASURING BLOOD INFORMATION**

(57) Blood information such as hemolysis (a plasma-free hemoglobin concentration) and a blood coagulation level (thrombus) can be obtained by extracting only reflected light in a plasma layer, and non-invasively and continuously obtaining information only on a plasma component independently of a hematocrit without separating blood components by a mechanical or chemical process.

First measurement light 30 is caused to be incident on a boundary surface between blood 10 flowing through a flow cell 40 formed of a transparent material having a different refractive index from plasma (layer) 12 in the blood 10 and the flow cell 40, from an oblique direction at an angle smaller than 90 degrees. Reflected light 32 regularly reflected at the boundary surface between the flow cell 40 and the blood 10 is subjected to spectrometry. Information on a plasma component (a refractive index Np of plasma) is obtained from an absorption spectrum measured.

Fig. 6

**EP 2 793 015 A1**

**Description**

TECHNICAL FIELD

The present invention relates to a method and a device for measuring blood information. In particular, the present invention relates to a method and a device for measuring blood information such as hemolysis (a plasma-free hemoglobin concentration) and a blood coagulation level (thrombus) wherein the method and the device can non-invasively and continuously obtain information on only a plasma component without relying on a hematocrit.

BACKGROUND ART

It is desired to non-invasively and continuously measure hemolysis and a blood coagulation level of the blood which is guided outside the living body through an artificial circulation circuit. Especially, although hemoglobin monitoring in dialysis is important as an index for observing water removal efficiency, currently used continuous hemoglobin monitors are not reliable.

Also, there is a risk of blood coagulation in all blood circulation system devices. Under such circumstances, extraction of information on a plasma component by light to continuously monitor anticoagulant agent effects and plasma-free hemoglobin is an essential technique to achieve a low-invasive treatment that does not require frequent blood collection, and to further achieve a treatment that requires less work burden for both patients and medical professionals.

As a known technique of measuring blood information, Patent Literature 1 discloses a particle analysis device that obtains characteristic parameters such as form information and light absorption information of particles (blood cells, cells and the like) contained in a sample liquid such as blood and urine from the light having passed through a flow cell.

Patent Literature 2 discloses a technique of measuring a concentration of total hemoglobin or red blood cells in a bloodstream by disposing a transmitted light sensor and a scattered light sensor to be orthogonal to each other so that the transmitted light sensor receives light along a transmission path running through a cuvette while the scattered light sensor receives light having scattered at an angle of 90 degrees with respect to the transmission path, and obtaining a ratio between scattered signals and transmitted signals.

Patent Literature 3 discloses a spectrophotometric analysis technique of blood in which a transmitted light sensor and a scattered light sensor are disposed in parallel to each other.

Patent Literature 4 discloses a blood coagulation analysis device that obtains, at a predetermined time interval, a scattered light amount value from a specimen to which a predetermined reagent is added, and that detects a coagulation endpoint on the basis of a time-dependent change in the scattered light amount value.

Patent Literature 5 discloses a blood coagulation measuring device that receives scattered light from a blood sample, and that measures saturation in a time-dependent change of a scattered light amount after addition of a coagulation reagent to the blood sample, to calculate a coagulation time.

The inventors have proposed a Monte Carlo simulation method for light propagation in blood in Non-Patent Literatures 1 and 2.

PRIOR ART DOCUMENT

PATENT LITERATURE

Patent Literature 1: Japanese Patent Application Laid-Open No. Hei. 6-186156
Patent Literature 2: Japanese Translation of PCT International Application Publication No. 2002-531824
Patent Literature 3: Japanese Patent Application Laid-Open No. Hei. 6-38947
Patent Literature 4: Japanese Patent Application Laid-Open No. 2010-210759
Patent Literature 5: Japanese Patent Application Laid-Open No. Hei. 10-123140

NON-PATENT LITERATURE

Non-Patent Literature 1: D. Sakota et al., Journal of Biomedical Optics, vol. 15(6), 065001(14 pp), 2010
Non-Patent Literature 2: D. Sakota, S. Takatani, "Newly developed photon-cell interactive Monte Carlo (pciMC) simulation for non-invasive and continuous diagnosis of blood during extracorporeal circulation support," Proc. SPIE 8092, 80920Y, 1-8 (2011)

## SUMMARY OF THE INVENTION

### PROBLEMS TO BE SOLVED BY THE INVENTION

The optical properties of blood depend on a volume of red blood cells MCV (a particle volume), a hemoglobin concentration in red blood cells MCHC (a particle refractive index), a hematocrit HCT (a particle density), and a plasma refractive index Np (a refractive index of solvents other than particles). Therefore, light propagation in blood can be considered as a function of these variables. However, it has been conventionally impossible to non-invasively and continuously measure information Np on a plasma component in blood.

The present invention has been made for solving the above-mentioned conventional problems. An object of the present invention is to enable non-invasive and continuous measurement of information on a plasma component in blood without separating blood components by a mechanical or chemical process.

### MEANS FOR SOLVING THE PROBLEMS

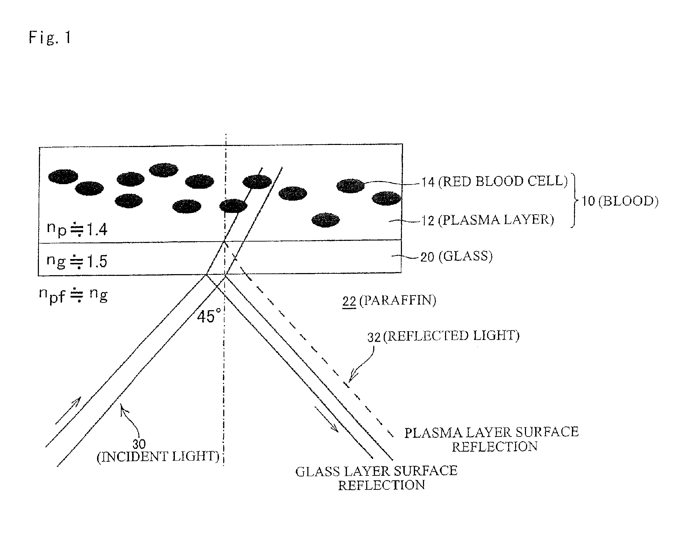

The inventors have found that, as schematically shown in FIG. 1, information on a plasma layer (also merely referred to as plasma) 12 in blood 10 can be obtained when first measurement light (also referred to as incident light) 30 is caused to be incident from the inside of paraffin 22 having approximately the same refractive index as glass 20 on a boundary surface between the glass 20 and the blood 10 flowing through a flow cell formed of the glass 20 that is a transparent material having a different refractive index from the plasma layer 12 in an oblique direction at 45 degrees, and light (also referred to as reflected light) 32 regularly reflected (here, totally reflected) at the boundary surface between the glass 20 and the blood 10 is subjected to spectrometry. That is, the refractive index Np of the plasma is a complex number represented by Np = Np-r + i*Np-i (i is an imaginary unit) wherein substantially constantly Np-r = 1.35 in general, and the refractive index Ng of the glass 20 is 1.5. Accordingly, the condition for total reflection is satisfied. Here, Np-i is related to light absorption, which can be obtained by determining an absorption spectrum. Np-i varies depending on protein contained in the plasma and the blood coagulation state. That is, NP-i varies depending on the chemical composition of the plasma. The principle of the spectrum measurement is that when reflection occurs at the boundary between the glass and the plasma layer, the boundary causes evanescent light to be generated. The interaction between the evanescent light and the substance (the plasma layer) reduces light intensity. The information on a plasma component can be obtained by measuring the reduction level for each wavelength thereof using a spectrophotometer. In the above measuring method, the light does not pass through the blood 10 that is an object. Therefore, Np can be measured without basically relying on blood cells.

However, when blood cells suspended in the plasma collide with the boundary between the glass and the plasma layer, spectral information on the blood cells is also contained. This becomes noise in measuring Np. To address this concern, measurement is performed in a state where blood is flowing through the flow cell. Hydrodynamically, microparticles in a solvent have a nature of gathering in the center of the flow cell where the flow rate is high. Accordingly, an increase of a flow rate in the flow cell significantly reduces blood cells moving toward the wall boundary, enabling elimination of noise. However, in order to inhibit the flow from becoming turbulent, the flow rate is desirably set at a Reynolds number Re of not higher than 2000 (for example, 5.28 L/min or less).

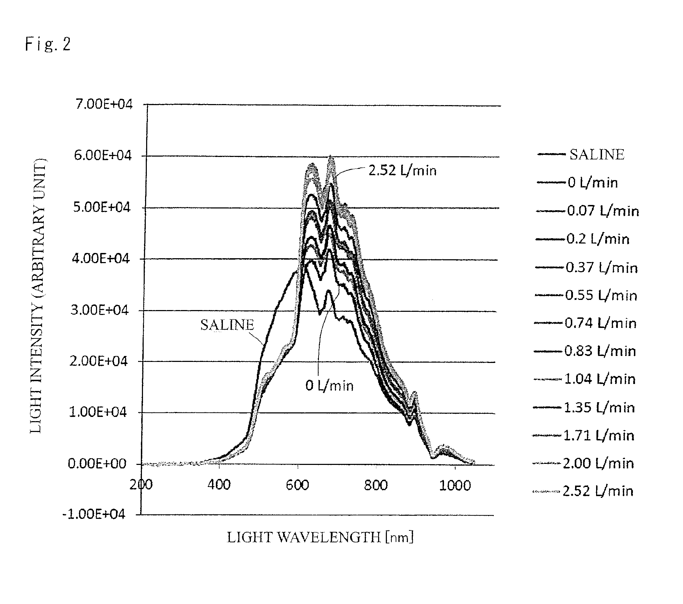

FIG. 2 shows a spectral change for each flow rate as the flow rate of a circulation circuit is changed. It can be seen that an increase of the flow rate increases received light intensity that is reflected light intensity.

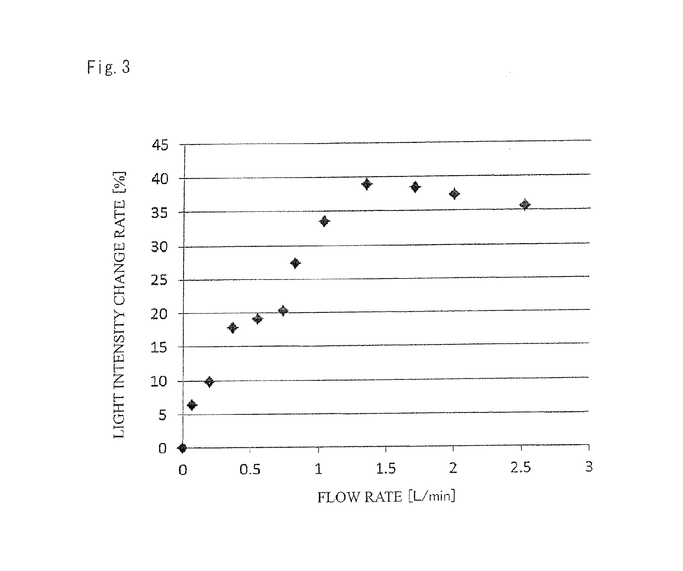

The waveform of FIG. 2 is integrated, and FIG. 3 shows a spectral change rate with respect to the flow rate of 0 L/min. This indicates that the change decreases and becomes substantially constant at the flow rate of 1.35 L/min or more. Strictly speaking, the change is caused, but the deviation thereof is as small as 1.47%. Therefore, there is virtually no problem in the measurement.

Therefore, at the flow rate of 1.35 L/min or more, the orientation of the distribution of red blood cells in blood becomes stable. Accordingly, a spectrum becomes stable without depending on the flow rate, thereby facilitating the measurement. Alternatively, once the relationship between the flow rate and the spectral change rate is previously checked, correction can be performed, and measurement can be performed at any flow rate.

The horizontal axis of FIG. 3 is presently the flow rate, which can be divided by the cross-sectional area of the flow cell so as to be converted into an average flow velocity.

Furthermore, when the viscosity and the density of blood are taken into account, the Reynolds number Re defined by the following formula can be calculated:

$$\mathrm{Re} = \mathrm{UD}/(\mu/\rho) \quad \cdots \quad (1).$$

Here, U is a characteristic flow velocity [m/sec], D is a characteristic length [m], $\mu$ is a fluid viscosity [Pa·s], and $\rho$ is a fluid density [kg/m$^3$]. The Reynolds number Re indicates the ratio between viscous forces and inertial forces, and a larger Re means stronger inertial forces. Viscous forces mean frictional resistance caused by viscosity that fluid itself has when the fluid moves (flows). The viscous forces become forces of being dragged by the neighboring fluid elements to move in a similar manner to the fluid elements. That is, in a flow field with a certain flow distribution, the viscous forces express forces permitting a fluid to move along the flow line. Therefore, as the Reynolds number Re is lower (viscous forces are higher), the flow is inhibited from becoming turbulent and becomes a laminar flow along the flow line. On the other hand, inertial forces express the opposite. The inertial forces mean inertia generated by a mass of a moving fluid, and express forces to move against the neighboring fluid elements. This means that as the inertial forces are stronger, the fluid freely behaves without following the viscous forces. Therefore, as the Reynolds number Re is higher (the inertial forces are higher), the flow is unlikely to become constant, and becomes a turbulent flow that is in chaos. A rough standard of transition from a laminar flow to a turbulent flow is said to be Re > 2000.

The Reynolds number Re, which is a dimensionless measure to express how orderly a fluid behaves, is used as a similarity rule of a flow. For example, when a flow inside a tube is considered, the pattern of the flow is the same as long as the Reynolds number Re is the same, even when the tube diameter, or the viscosity and the density of the fluid vary. Therefore, even when the size of the flow cell varies (the shape is similar), and even when the density and the viscosity of blood vary, the measurement comes to be similarly performed as long as the condition is satisfied in terms of the Reynolds number Re. Therefore, the measurement condition itself can be exactly expressed by numerical values.

Then, the Reynolds number Re at 1.35 L/min is calculated. The characteristic length D of the formula (1) is a tube diameter in the case of a tube. The present flow cell has a cross section of a square. In this case, the characteristic length D is the length of a side of the square, that is D = 10 × 10$^{-3}$ m. The characteristic flow velocity U is, according to:

$$1.35 \text{ [L/min]} = 1360 \text{ [cm}^3\text{/min]}$$

$$= 22.67 \text{ [cm}^3\text{/sec]}$$

$$= 22.67 \times 10^3 \text{ [mm}^3\text{/sec]},$$

$$U = (22.67 \times 10^3 \text{ [mm}^3\text{/sec]})/(100 \text{ [mm}^2\text{]})$$

$$= 226.7 \text{ [mm/sec]}$$

$$= 0.2267 \text{ [m/sec]}.$$

Viscosity $\mu$, and density $\rho$ vary depending on a hematocrit and a hemoglobin amount of blood. Therefore, a typical value is employed here. Based on $\rho$ = 1.06 × 10$^3$ [kg/m$^3$] and $\mu$ = 4.7 × 10$^{-3}$ [Pa·sec] in blood of an adult male, the Reynolds number Re is

$$Re = UD/(\mu/\rho) = 511.2.$$

Thus, at Re = 511.2 or more, the spectrum becomes stable, and measurement can be easily performed.

When the measurement condition for spectrometry is determined by the Reynolds number Re, the same condition can be set even when the fluid varies, as long as the Reynolds number Re is the same. Therefore, the Reynolds number Re can be considered as the most suitable parameter to determine the condition in a fluid. However, since the viscosity and the density of blood are not actually measured in each case, the measurement condition may be defined by the flow velocity U without problems.

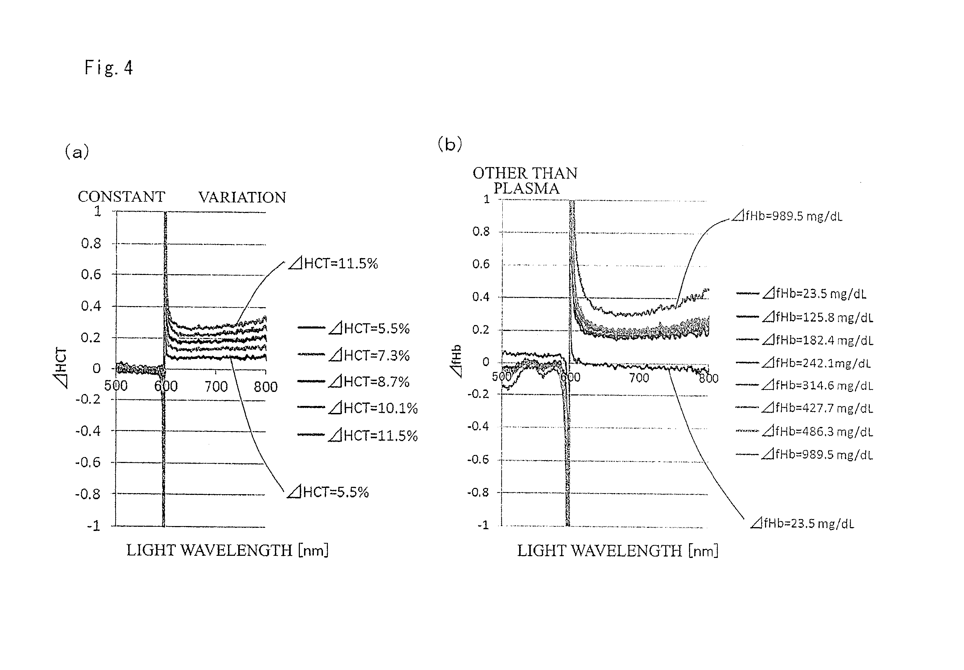

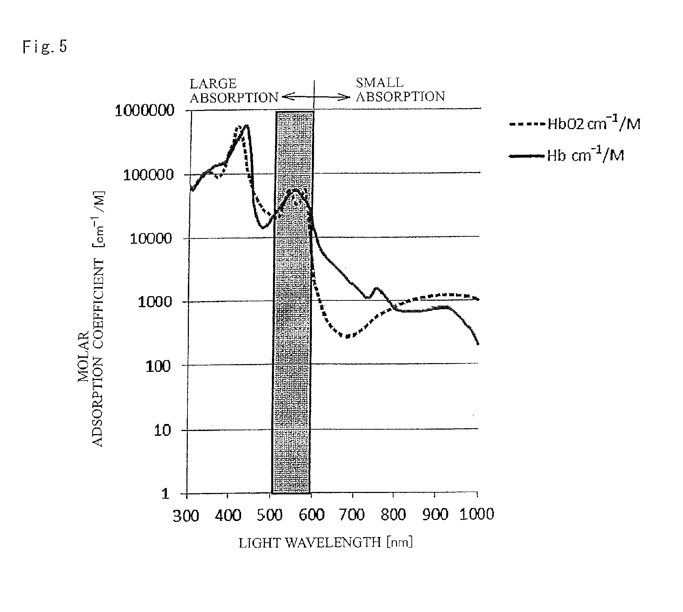

The wavelength of light colliding with the boundary surface is desirably 600 nm or shorter, more preferably 500 to 600 nm. This is because while a varied hematocrit HCT hardly causes the spectrum to be changed at a wavelength of 500 nm to 600 nm as indicated by a differential spectrum ΔHCT of HCT in FIG. 4(a), hemolysis is characteristic as indicated by a differential spectrum ΔfHb of a plasma-free hemoglobin fHb in FIG. 4(b). In this case, a characteristic of the light absorption property of hemoglobin Hb depending on a plasma-free hemoglobin fHb is obtained, and reflection spectrometry at the plasma layer boundary can be performed in this wavelength range. On the other hand, in the wavelength range of 600 nm to 800 nm as shown in FIG. 5, absorption by the hemoglobin Hb is small. Accordingly, scattered light by red blood cells is detected, and as shown in FIG. 4, the spectrum changed in accordance with the change in the hematocrit and the hemolysis.

The incident angle is not limited to 45 degrees or smaller in some material of the flow cell. Also, total reflection is not mandatory. Furthermore, the light wavelength may be 600 nm or longer.

The present invention has been made on the basis of the knowledge as described above, the above-described problems can be solved by causing first measurement light to be incident on a boundary surface between blood flowing through a flow cell formed of a transparent material having a different refractive index from plasma and the flow cell, from an oblique direction at an angle smaller than 90 degrees; and performing spectrometry of light regularly reflected at the boundary surface between the flow cell and the blood, to obtain information on a plasma component from an absorption spectrum measured.

Here, the information on a plasma component can be a refractive index of the plasma.

Also, the reflected light can be totally reflected light from the boundary surface.

Also, the Reynolds number or the flow rate of the blood flowing through the flow cell can be set to fall within a predetermined range (for example, 511 or more and 2000 or less in terms of the Reynolds number Re, 1.35 L/min or more and 5.28 L/min or less in terms of the flow rate).

Also, the wavelength of the first measurement light to be incident on the boundary surface can be 600 nm or shorter.

Also, an incident angle of the first measurement light with respect to the boundary surface can be 45 degrees or smaller.

Information on blood cells can be obtained by: performing spectrometry of transmitted light that passes through a blood flow path of a flow cell formed of a transparent material when second measurement light is caused to be incident perpendicularly to a side wall parallel to the blood flow path of the flow cell and that exits from the opposite side to obtain information on blood cells and a plasma component from an absorption spectrum thereof; and comparing the obtained information with the information on the plasma component obtained in the above-described method.

Also, the first measurement light may be caused to be incident on one slope of the side walls of the flow cell having a trapezoid shape including a bottom on the blood flow path side to measure the plasma component, and at the same time the second measurement light may be caused to be incident perpendicularly to the side wall parallel to the blood flow path of the same flow cell to measure the blood cells and the plasma component described above.

Also, the measurement of a plasma component and the measurement of blood cells and a plasma component can be alternately performed.

The present invention has also solved the above-described problems with a device for measuring blood information. The measuring device includes: a flow cell formed of a transparent material having a different refractive index from plasma and including side walls of a blood flow path, one of the side walls having a pair of slopes outside; a first light source for causing first measurement light to be incident on one slope of the flow cell; and first spectrometry means for performing spectrometry of reflected light that is reflected at a boundary surface between the blood flow path of the flow cell and blood and that exits from the other slope of the flow cell to obtain information on a plasma component from an absorption spectrum measured.

Here, the transparent material can be glass, plastics and/or paraffin.

The measuring device may further include: a second light source for causing second measurement light to be incident perpendicularly to a side wall parallel to the blood flow path of the flow cell; second spectrometry means for performing spectrometry of transmitted light that passes through the blood flow path of the flow cell and that exits from the opposite side to obtain information on blood cells and a plasma component from an absorption spectrum measured; and calculation means for comparing the information on blood cells and a plasma component obtained in the second spectrometry means with the information on a plasma component obtained in the first spectrometry means to obtain information on blood cells.

The first and/or second light sources can be a white light source.

Also, one of the side walls of the flow cell may have a trapezoid shape with a bottom on the blood flow path side, and the flow cell for obtaining information on a plasma component and the flow cell for obtaining information on blood cells and a plasma component may be made common.

Alternatively, the flow cell for obtaining information on a plasma component and the flow cell for obtaining information on blood cells and a plasma component may be independently provided.

ADVANTAGEOUS EFFECTS OF THE INVENTION

According to the present invention, blood information such as hemolysis and a blood coagulation level can be obtained by non-invasively and continuously measuring information on only a plasma component independently of a hematocrit without separating blood components by a mechanical or chemical process. Therefore, hemolysis and thrombus can be non-invasively and continuously measured, and the pharmaceutical effect of anticoagulant agents and the damage level of blood cells can be grasped.

BRIEF DESCRIPTION OF THE DRAWINGS

FIG. 1 is a schematic diagram for illustrating the principle of the present invention;
FIG. 2 is similarly a diagram showing an example of the relationship between the flow rate and the spectrum;
FIG. 3 is similarly a diagram showing the change rate of the spectrum with respect to the flow rate shown in FIG. 2;
FIG. 4 is similarly diagrams each showing a differential spectrum of (a) a hematocrit HCT or (b) a plasma-free hemoglobin fHb for comparison;
FIG. 5 is similarly a diagram showing the light absorption property of hemoglobin Hb;
FIG. 6 is a cross-sectional diagram showing the configuration of a first embodiment of the present invention;
FIG. 7 is a cross-sectional diagram showing the configuration of a second embodiment of the present invention; and
FIG. 8 is a schematic diagram showing the configuration of a third embodiment of the present invention.

MODE FOR CARRYING OUT THE INVENTION

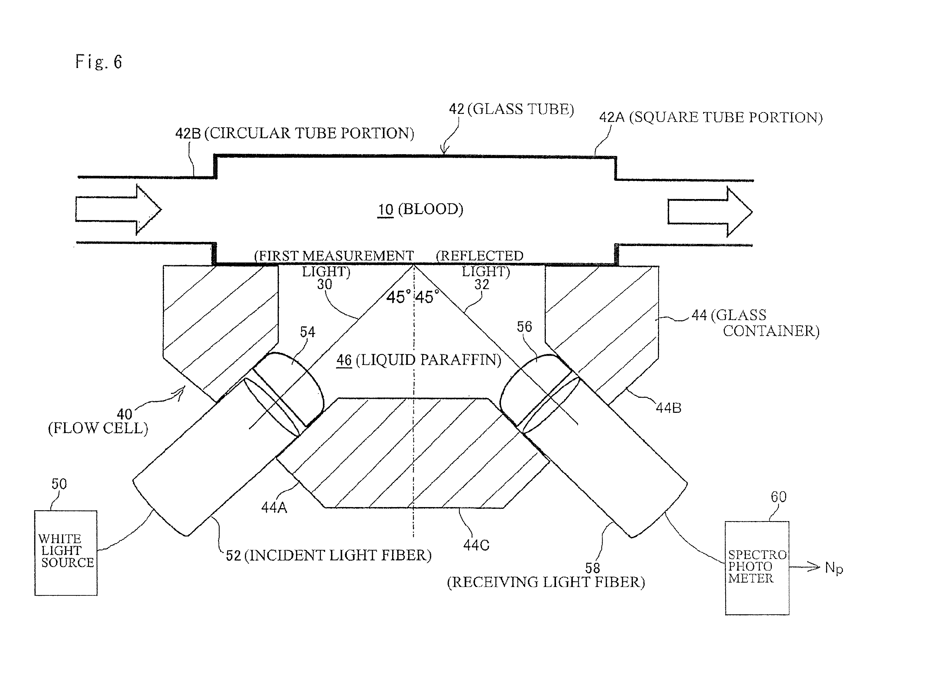

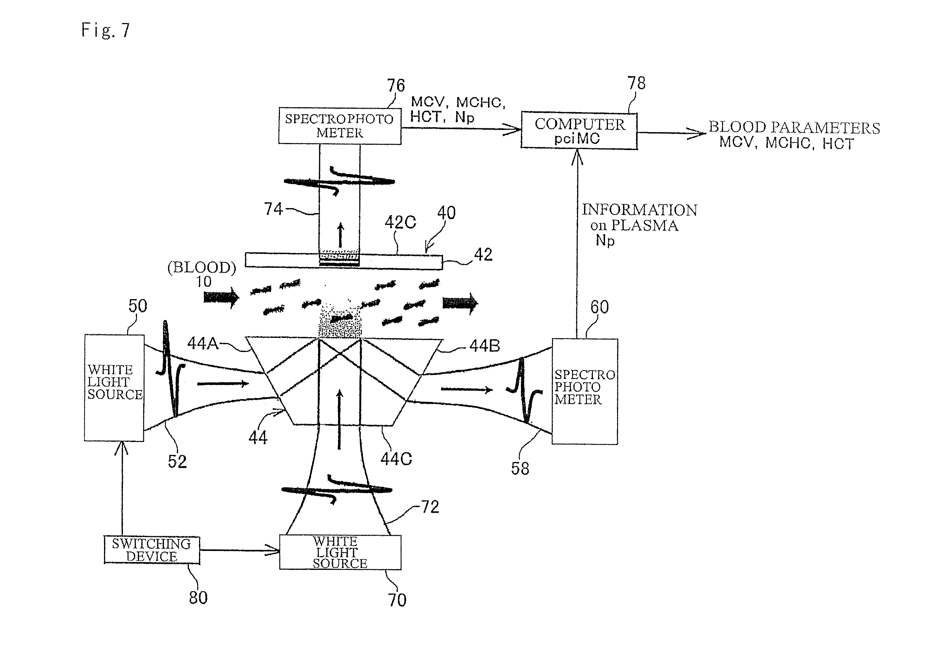

Embodiments of the present invention are described in detail below with reference to the drawings.
As shown in FIG. 6, a first embodiment of the present invention includes: a flow cell 40 constituted by a glass tube 42 that has a cross section of a square and is formed into a tube shape and that constitutes a blood flow path, a glass container 44 that is fixed to one side wall (a lower side wall in the diagram) of the glass tube 42 and that has a trapezoid shape, and a liquid paraffin 46 filled in the glass container 44; a white light source 50; an incident light fiber 52 for causing white light generated by the white light source 50 to be incident on one slope (a slope on the left side in the diagram) 44A of the glass container 44 through a collimator lens 54 as first measurement light (incident light) 30; a receiving light fiber 58 for detecting reflected light 32 that is regularly reflected at a boundary surface between the blood 10 and the glass tube 42 and exits from the other slope (a slope on the right side in the diagram) 44B of the glass container 44 through a collimator lens 56; and a first spectrophotometer 60 for performing spectrometry of the reflected light obtained by the receiving light fiber 58 to obtain information Np on a plasma component from an absorption spectrum measured.
For example, the glass tube 42 has a glass wall thickness of 1.25 mm, and includes a square tube portion 42A with a cross section of a square of 10 mm $\times$ 10 mm and a length of 42.5 mm, and circular tube portions 42B on an inlet side and an outlet side with a diameter of 4.5 mm and a length of 15 mm. Also, a space in which the liquid paraffin 46 is filled is shaped into a cylinder with an inner diameter of 30 mm and a depth of 15 mm.
As the white light source 50, for example, a halogen white light source having a wavelength of 300 nm to 1100 nm can be used.
An operation will be described below.
White light guided through the incident light fiber 52 is caused to be incident on a side surface of the glass container 44 of the flow cell 40. The angle formed between the incident axis and the glass side surface is determined as such an angle that allows the light to pass through the glass and be totally reflected at the boundary between the glass and the plasma layer. The reflected light 32 is guided to the first spectrophotometer 60 through the receiving light fiber 58. Then, an absorption spectrum is determined, so as to determine a refractive index Np-i related to a light absorption rate.
Next, a second embodiment of the present invention is described.
As shown in FIG. 7, the present embodiment further includes: a second white light source 70; a second spectrophotometer 76 for causing white light to be incident through an incident light fiber 72 on a side wall (a top surface on the lower side in the diagram) 44C parallel to the blood flow path (the glass tube 42) of the flow cell 40 similar to that in the first embodiment and receiving transmitted light that passes through the blood flow path of the flow cell 40 and exits from an opposite side 42C thereto through a receiving light fiber 74 to obtain information on blood cells and a plasma component MCV, MCHC, HCT and Np; and a computer 78 for comparing the information on blood cells and a plasma component obtained by the second spectrophotometer 76 with the information Np on a plasma component obtained by the first spectrophotometer 60 according to the first embodiment, to obtain blood cell information MCV, MCHC and HCT.
In the second embodiment, the white light guided through the incident light fiber 72 is perpendicularly incident on the top surface 44C of the trapezoid of the glass container 44. The light passes through the glass, and further passes through the blood. Then, the transmitted light is received by the receiving light fiber 74 disposed on the opposite surface 42C to the incident side of the flow cell, and guided to the second spectrophotometer 76 to measure a light absorption spectrum. Unlike the first embodiment, in the case of the above measurement, light is propagated in blood. Accordingly, the light is absorbed and scattered mainly by red blood cells. Since the representative absorber is hemoglobin, a spectrum having a wavelength of 600 nm or longer, which is less absorbed by hemoglobin, is used. Furthermore, to address the varied absorption by hemoglobin depending on a blood oxygen saturation, a received light intensity at an isosbestic wavelength (a wavelength at which absorption does not depend on an oxygen saturation) of 805 nm is set as a standard. That is, an absorption spectrum in the range of $\pm 30$ nm of 805 nm (775 nm to 835 nm) where there is next to no wavelength dependence with respect to scattering is next used.

Meanwhile, this measurement state is input to the computer 78 to perform the Monte Carlo simulation (photon-cell interactive Monte Carlo simulation: pciMC) of light propagation in blood which has been proposed by the inventors in Non-Patent Literatures 1 and 2. In this simulation, input parameters of blood are MCV, MCHC, HCT and Np. As Np, the value obtained according to the first embodiment is input. As each of other three variables, an appropriate value is input as an initial value. As a range that sufficiently contains a clinically possible range, for example, the range of MCV can be 70 to 110 fL, the range of MCHC can be 25 to 40 g/dL, and the range of HCT can be 20 to 60%. Also, the wavelength is set in the range of 775 to 835 nm, and the pciMC simulation is performed to obtain an absorption spectrum. An inverse problem is performed to explore MCV, MCHC and HCT that are input values of the pciMC where the spectrum obtained in the simulation coincides with the actually measured spectrum (the inverse Monte Carlo method). The actually performed method includes previously simulating the whole range of the above-described input parameters to build a database of the simulation, and exploring MCV, MCHC and HCT that each coincide with the measurement result in the database. Thus, the calculation cost can be minimized.

In the second embodiment, the side surface of the trapezoid-type cell is irradiated with the light to allow the light to be totally reflected at the boundary. Therefore, scattering by the red blood cells is theoretically 0. Thus, compared to the first embodiment, noise is reduced, and pure information on a refractive index of plasma can be extracted. Therefore, the measurement can be performed with higher accuracy than in the first embodiment.

In the second embodiment, two light incident locations and two light receiving locations are provided to measure both the plasma component and the blood cell component. To prevent the two types of light from interfering with each other under such circumstances, a switching device 80 may be provided so that the white light sources 50 and 70 are alternately switched on/off to allow for alternate light illumination for plasma measurement and for blood cell measurement. The switching frequency may be set at approximately 1 Hz. Blood cell output calculation may be performed during the plasma measurement, and plasma output calculation is performed during the blood cell measurement. Thus, both measurement values can be output without intermittence at a switching frequency interval.

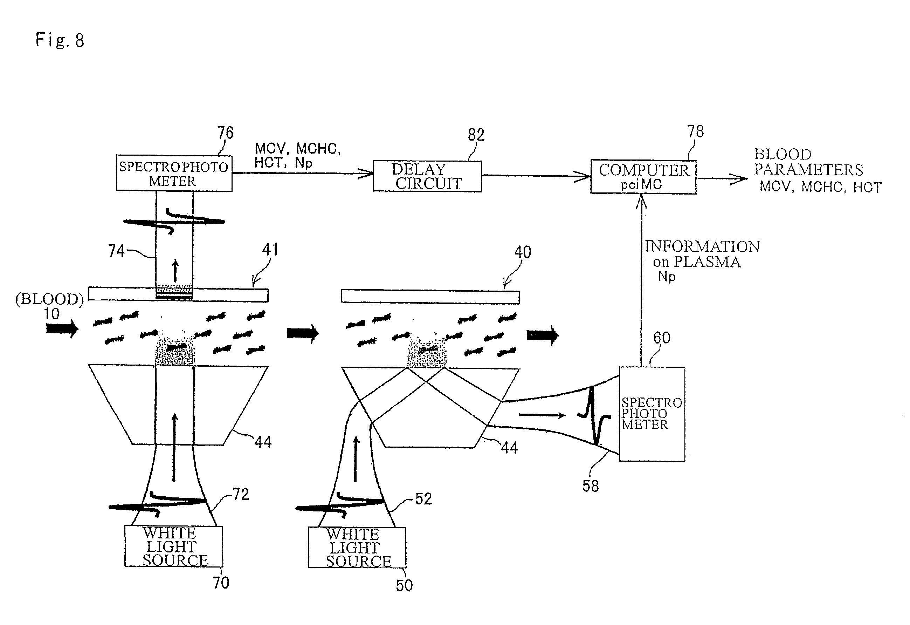

Alternatively, as in a third embodiment shown in FIG. 8, a flow cell 40 for measuring plasma and a flow cell 41 for measuring a blood cell may be separately and tandemly disposed, so as to continuously perform the plasma measurement and the blood cell measurement. In this case, a delay circuit 82 that performs delaying in accordance with the flow rate of blood may be provided to obtain information of the same blood part. Here, a delay time can be changed in accordance with a measured flow rate of blood, or can be constant while the flow rate of blood is set constant. The flow cell 41 for measuring a blood cell may not have a trapezoid shape, and may have a simple cylinder shape.

Although the cross section of the glass tube 42 is set at 1 cm$^2$ in the above-mentioned embodiment, it may be smaller than that when the flow rate of blood is low. The light source is also not limited to the halogen white light source.

INDUSTRIAL APPLICABILITY

The present invention can obtain information only on the plasma component to be obtained noninvasively and continuously, and can be used for measurement of blood information, such as hemolysis (a concentration of plasma free hemoglobin) or degree of blood coagulation (a thrombus).

The disclosure of the specification, drawings, and claims of Japanese Patent Application No. 2012-028231 filed on February 13, 2012 is incorporated herein by reference in its entirety.

REFERENCE SIGNS LIST

| | |
|---|---|
| 10 | Blood |
| 12 | Plasma (layer) |
| 14 | Red blood cell |
| 30 | Incident light (first measurement light) |
| 32 | Reflected light |
| 40 | Flow cell |

| 42 | Glass tube (blood flow path) |
|---|---|
| 44 | (Trapezoid-shaped) glass container |
| 44A, 44B | Slope |
| 44C | Top surface |
| 46 | Liquid paraffin |
| 50, 70 | White light source |
| 52, 72 | Incident light fiber |
| 58, 74 | Receiving Light fiber |
| 60, 76 | Spectrophotometer |
| 78 | Computer |
| 80 | Switching device |
| 82 | Delay circuit |

**Claims**

1. A method for measuring blood information, comprising:

   causing first measurement light to be incident on a boundary surface between blood flowing through a flow cell formed of a transparent material having a different refractive index from plasma and the flow cell, from an oblique direction at an angle smaller than 90 degrees; and
   performing spectrometry of light regularly reflected at the boundary surface between the flow cell and the blood, to obtain information on a plasma component from an absorption spectrum measured.

2. The method for measuring blood information according to claim 1, wherein the information on a plasma component is a refractive index of the plasma.

3. The method for measuring blood information according to claim 1 or 2, wherein the reflected light is totally reflected light from the boundary surface.

4. The method for measuring blood information according to any of claims 1 to 3, wherein a Reynolds number or a flow rate of the blood flowing through the flow cell is set to fall within a predetermined range.

5. The method for measuring blood information according to any of claims 1 to 4, wherein a wavelength of the first measurement light to be incident on the boundary surface is 600 nm or shorter.

6. The method for measuring blood information according to any of claims 1 to 5, wherein an incident angle of the first measurement light with respect to the boundary surface is 45 degrees or smaller.

7. A method for measuring blood information, comprising:

   performing spectrometry of transmitted light that passes through a blood flow path of a flow cell formed of a transparent material when second measurement light is caused to be incident perpendicularly to a side wall parallel to the blood flow path of the flow cell and that exits from the opposite side to obtain information on blood cells and a plasma component from an absorption spectrum thereof; and
   comparing the obtained information with the information on the plasma component obtained in the method of any of claims 1 to 6 to obtain information on blood cells.

8. The method for measuring blood information according to claim 7, wherein the first measurement light is caused to be incident on one slope of the side walls of the flow cell having a trapezoid shape including a bottom on a blood flow path side to measure the plasma component according to any of claims 1 to 6, and the second measurement light is caused to be incident perpendicularly to the side wall parallel to the blood flow path of the same flow cell to measure the blood cells and the plasma component according to claim 7.

9. The method for measuring blood information according to claim 8, comprising alternately performing measuring the plasma component according to any of claims 1 to 6 and measuring the blood cells and the plasma component according to claim 7.

**10.** A device for measuring of blood information, comprising:

a flow cell formed of a transparent material having a different refractive index from plasma and including side walls of a blood flow path, one of the side walls having a pair of slopes outside;
a first light source for causing first measurement light to be incident on one slope of the flow cell; and
first spectrometry means for performing spectrometry of reflected light that is reflected at a boundary surface between the blood flow path of the flow cell and blood and that exits from the other slope of the flow cell to obtain information on a plasma component from an absorption spectrum measured.

**11.** The device for measuring blood information according to claim 10, wherein the transparent material is glass, plastics and/or paraffin.

**12.** The device for measuring blood information according to claim 10 or 11, further comprising:

a second light source for causing second measurement light to be incident perpendicularly to a side wall parallel to the blood flow path of the flow cell;
second spectrometry means for performing spectrometry of transmitted light that passes through the blood flow path of the flow cell and that exits from the opposite side to obtain information on blood cells and a plasma component from an absorption spectrum measured; and
calculation means for comparing the information on blood cells and a plasma component obtained in the second spectrometry means with the information on a plasma component obtained in the first spectrometry means to obtain information on blood cells.

**13.** The device for measuring blood information according to any of claims 10 to 12, wherein the first and/or second light sources are a white light source.

**14.** The device for measuring blood information according to any of claims 10 to 13, wherein one of the side walls of the flow cell has a trapezoid shape with a bottom on the blood flow path side, and the flow cell for obtaining information on a plasma component and the flow cell for obtaining information on blood cells and a plasma component are made common.

**15.** The device for measuring blood information according to any of claims 10 to 13, wherein the flow cell for obtaining information on a plasma component and the flow cell for obtaining information on blood cells and a plasma component are independently provided.

Fig. 1

$n_p \fallingdotseq 1.4$

$n_g \fallingdotseq 1.5$

$n_{pf} \fallingdotseq n_g$

14 (RED BLOOD CELL)

12 (PLASMA LAYER)

10 (BLOOD)

20 (GLASS)

22 (PARAFFIN)

32 (REFLECTED LIGHT)

45°

30
(INCIDENT LIGHT)

PLASMA LAYER SURFACE
REFLECTION

GLASS LAYER SURFACE
REFLECTION

Fig. 2

Fig. 3

Fig. 4

(a)

(b)

EP 2 793 015 A1

Fig. 5

Fig. 6

42 (GLASS TUBE)  42A (SQUARE TUBE PORTION)

42B (CIRCULAR TUBE PORTION)

10 (BLOOD)

(FIRST MEASUREMENT  (REFLECTED
LIGHT)  LIGHT)
30  32
45° 45°

44 (GLASS
CONTAINER)

44B

46 (LIQUID PARAFFIN)

54  56

40
(FLOW CELL)

44A

44C

50

WHITE
LIGHT
SOURCE

52 (INCIDENT LIGHT FIBER)

58
(RECEIVING LIGHT FIBER)

60

SPECTRO
PHOTO
METER

→ Np

EP 2 793 015 A1

Fig. 7

EP 2 793 015 A1

Fig. 8

SPECTRO PHOTO METER 76

MCV, MCHC, HCT, Np

DELAY CIRCUIT 82

COMPUTER pciMC 78

BLOOD PARAMETERS MCV, MCHC, HCT

74

41

40

(BLOOD) 10

INFORMATION on PLASMA Np

44

44

60

SPECTRO PHOTO METER

58

72

52

WHITE LIGHT SOURCE

WHITE LIGHT SOURCE

70

50

EP 2 793 015 A1

## INTERNATIONAL SEARCH REPORT

| International application No. |
| --- |
| PCT/JP2013/053321 |

A.　CLASSIFICATION OF SUBJECT MATTER
*G01N21/27*(2006.01)i

According to International Patent Classification (IPC) or to both national classification and IPC

B.　FIELDS SEARCHED

Minimum documentation searched (classification system followed by classification symbols)
G01N21/27

Documentation searched other than minimum documentation to the extent that such documents are included in the fields searched

| | | | |
| --- | --- | --- | --- |
| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)

C.　DOCUMENTS CONSIDERED TO BE RELEVANT

| Category* | Citation of document, with indication, where appropriate, of the relevant passages | Relevant to claim No. |
| --- | --- | --- |
| A | JP 9-311099 A (Hitachi, Ltd.), 02 December 1997 (02.12.1997), entire text; all drawings (Family: none) | 1–15 |
| A | JP 1-500928 A (Radiometer A/S), 30 March 1989 (30.03.1989), entire text; all drawings & WO 1988/001376 A1 | 1–15 |
| A | JP 2008-224524 A (Toshiba Corp.), 25 September 2008 (25.09.2008), entire text; all drawings & US 2008/0227126 A1 | 1–15 |

☒　Further documents are listed in the continuation of Box C.　　☐　See patent family annex.

| | |
| --- | --- |
| *　　Special categories of cited documents:<br>"A"　document defining the general state of the art which is not considered to be of particular relevance<br>"E"　earlier application or patent but published on or after the international filing date<br>"L"　document which may throw doubts on priority claim(s) or which is cited to establish the publication date of another citation or other special reason (as specified)<br>"O"　document referring to an oral disclosure, use, exhibition or other means<br>"P"　document published prior to the international filing date but later than the priority date claimed | "T"　later document published after the international filing date or priority date and not in conflict with the application but cited to understand the principle or theory underlying the invention<br>"X"　document of particular relevance; the claimed invention cannot be considered novel or cannot be considered to involve an inventive step when the document is taken alone<br>"Y"　document of particular relevance; the claimed invention cannot be considered to involve an inventive step when the document is combined with one or more other such documents, such combination being obvious to a person skilled in the art<br>"&"　document member of the same patent family |

| Date of the actual completion of the international search<br>　　09 April, 2013 (09.04.13) | Date of mailing of the international search report<br>　　16 April, 2013 (16.04.13) |
| --- | --- |
| Name and mailing address of the ISA/<br>　　Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2013/053321

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2011-232137 A  (Nippon Telegraph and Telephone Corp.), 17 November 2011 (17.11.2011), entire text; all drawings (Family: none) | 1-15 |
| A | WO 2006/095615 A1  (Kuraray Co., Ltd.), 14 September 2006 (14.09.2006), entire text; all drawings & EP 1860443 A1 | 1-15 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI6186156 B
- JP 2002531824 PCT
- JP HEI638947 B

- JP 2010210759 A
- JP HEI10123140 B
- JP 2012028231 A

**Non-patent literature cited in the description**

- **D. SAKOTA et al.** *Journal of Biomedical Optics,* 2010, vol. 15 (6), 065001

- **D. SAKOTA ; S. TAKATANI.** Newly developed photon-cell interactive Monte Carlo (pciMC) simulation for non-invasive and continuous diagnosis of blood during extracorporeal circulation support. *Proc. SPIE 8092,* 2011, vol. 80920Y, 1-8